# FASCICULE DE BREVET EUROPEEN

(11) **EP 1 183 255 B1**
(45) Date de publication et mention de la délivrance du brevet: **05.03.2003**
(21) Numéro de dépôt: 00940473.2
(22) Date de dépôt: 08.06.2000
(51) Int. Cl.: C07D 471/08, A61K 49/10

(54) **COMPLEXES METALLIQUES DE POLYAMINOACIDES BICYCLIQUES, LEUR PROCEDE DE PREPARATION ET LEUR APPLICATION EN IMAGERIE MEDICALE**
BICYCLISCHE POLYAMIOSAURE METALLKOMPLEXE, VERFAHREN ZU IHRER HERSTELLUNG UND IHRE VERWENDUNG FÜR MEDIZINISCHE BILDERZEUGUNG
BICYCLIC POLYAMINOACID METAL COMPLEXES, METHOD FOR PREPARING SAME AND USE IN MEDICAL IMAGING

(30) Priorité: 09.06.1999 FR 9907283
(43) Date de publication de la demande: 06.03.2002
(73) Titulaire: GUERBET, 93420 Villepinte (FR)
(72) Inventeur: PORT, Marc, F-95170 Deuil la Barre (FR)
(74) Mandataire: Bernasconi, Jean Raymond
(86) Numéro de dépôt international: FR0001591
(87) Numéro de publication internationale: WO00075141

(56) Documents cités:
- EP-A- 0 352 218
- EP-A- 0 391 766
- EP-A- 0 438 206
- WO-A-93/11800
- WO-A-97/01359
- AIME S. ET AL: 'Synthesis and NMR Studies of Thre Pyridine-Containing Triaza Macrocyclic Triacetate Ligands and Their Complexes with Lanthanide Ions' INORG. CHEM. vol. 36, 1997, pages 2992 - 3000
- AIME S. ET AL: 'NMR relaxometric studies of Gd(III) complexes with heptadentate macrocyclic ligands' MAGN. RESON. CHEM. vol. 36, 1998, pages 200 - 208

## Description

La présente invention concerne des chelates métalliques, dérivés d'un macrocycle tétraazoté condensé avec un noyau pyridyle, leurs procédés de préparation et leur application en imagerie médicale.

L'utilisation en imagerie des chelates, avec un cation paramagnétique ou radioactif, de certains dérivés de ce macrocycle condensé a été proposée à différentes reprises. On peut se référer aux brevets EP-A-0438206, EP-A-0570575 et EP-A-0579802 qui décrivent des composés de formule 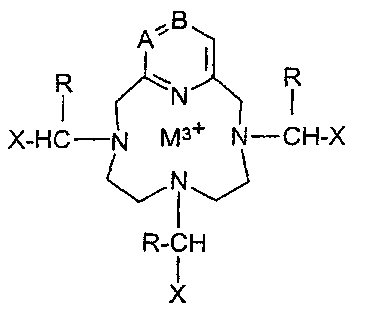 dans laquelle X peut être un groupement carboxylate ou phosphate et R un groupe alkyle, phényle ou l'un des R est un groupe formant une liaison avec une macromolécule biologique.

Parmi ces composés, celui pour lequel A = B = CH, R = H, X = CO₂⁻, M = Gd dénommé PCTA, a été l'objet d'études approfondies, décrites dans Inorganic Chemistry 36(14) 2992-3000 (1997) et Magn. Reson. Chem. 36 S200-208 (1998); les auteurs indiquent notamment que le PCTA est remarquable en ce qu'il a une relaxivité longitudinale r₁ particulièrement élevée puisqu'elle est environ "2 fois" celle des chelates de gadolinium utilisés comme produits de contraste en imagerie par résonance magnétique chez l'homme;

On sait que r₁ caractérise l'efficacité des produits paramagnétiques à générer un fort contraste des images et il est spécialement intéressant et inattendu, que les produits paramagnétiques de l'invention présentent des relaxivités r₁ de 10 à 15 fois supérieures à celle des composés commerciaux, non seulement pour un champ magnétique de 0,5 Tesla mais aussi de 1 Tesla, champ de la plupart des appareils d'imagerie actuels, et même de 1,5 Tesla, champ des appareils les plus performants.

Comme ces nouveaux chelates, outre leurs propriétés magnétiques favorables, sont stables in vitro et in vivo, notamment vis-à-vis d'une éventuelle décomplexation, qu'ils ont une faible osmolalité, un bon index thérapeutique et que selon la nature du groupe R ils peuvent présenter une excellente rémanence vasculaire ou une spécificité d'organe, ils seront avantageusement utilisés chez l'homme comme produits de contraste pour l'imagerie par résonance magnétique, ou en médecine nucléaire lorsque l'ion métallique est un radioélément.

La présente invention concerne les chelates métalliques du composé de formule 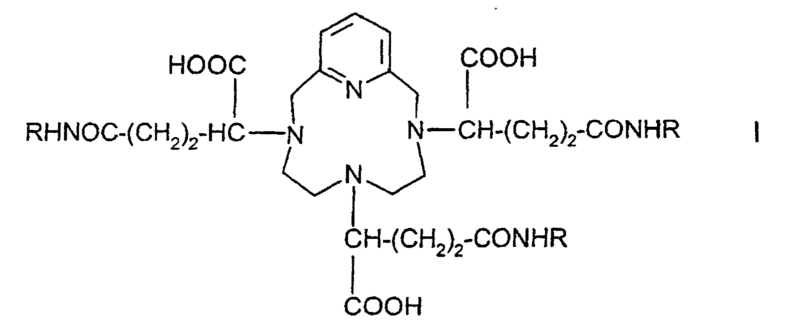 dans laquelle
R représente la formule 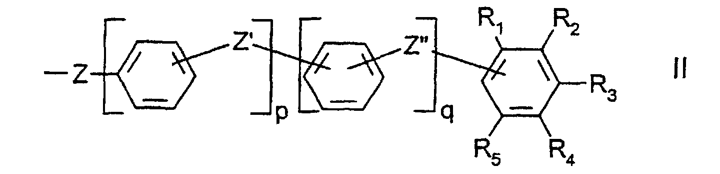 et
- Z est une liaison ou un groupe choisi parmi les groupes CH₂, CH₂-CO-NH ou (CH₂)₂-NH-CO,
- Z' est une liaison ou un groupe choisi parmi les groupes O, S, NQ, CH₂, CO, CO-NQ, NQ-CO, NQ-CO-NQ, CO-NQ-CH₂-CO-NQ,
- Z" une liaison ou un groupe choisi parmi les groupes CO-NQ, NQ-CO, CO-NQ-CH₂-CO-NQ,
- p et q sont des nombres entiers dont la somme vaut de 0 à 3,
- R₁, R₂, R₃, R₄ et R₅, indépendamment l'un de l'autre, sont choisis parmi les groupes H, Br, Cl, I, CO-NQ₁Q₂ et NQ₁-CO-Q₂ et Q₁ et Q₂ identiques ou différents étant H ou (C₁-C₆)alkyle éventuellement interrompu par un ou des atomes d'oxygène, et au moins l'un des R₁ à R₅ étant un groupe amido,
ou R₁, R₃ et R₅ sont indépendammant l'un de l'autre H, Br, Cl ou I, et
R₂ et R₄ représentent la formule 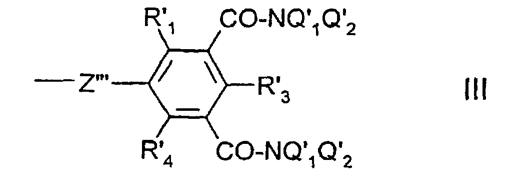 telle que Z"' est un groupe choisi parmi les groupes CO-NQ, CONQ-CH₂, CO-NQ-CH₂-CO-NQ, CO-NQ-(CH₂)₂-NQ-CO et NQ-CO-NQ et R'₁, R'₃ et R'₅, identiques ou différents, représentent H, Br, Cl ou I et Q'₁ et Q'₂, identiques ou différents, sont H ou (C₁-C₆)alkyle éventuellement interrompu par un ou des atomes d'oxygène,
- Q représente H ou (C₁-C₄)alkyle,
étant entendu que les groupes alkyles peuvent être éventuellement mono ou polyhydroxylés,

Les ions métalliques peuvent être paramagnétiques, tels que Gd³⁺, Fe³⁺, Tb³⁺, Mn²⁺, Dy³⁺, ou Cr³⁺, ou radioactifs, tels que ^{99m}Tc, ⁶⁷Ga ou ¹¹¹In; les ions qui forment des chelates moins stables, qui peuvent donner lieu à une transmétallation tels que Ca²⁺ ou Zn²⁺ font aussi partie de l'invention; les chelates paramagnétiques de Gd³⁺ et Mn²⁺ sont particulièrement adaptés à l'imagerie par résonance magnétique. On préfère tout particulièrement les chelates paramagnétiques de Gd³⁺.

On préfère pour une meilleure hydrophilie et biocompatibilité des composés, que les groupes R₁ à R₅ sur un même noyau phényle comportent ensemble de 6 à 20 OH, ou même que tout groupe CONQ₁Q₂ présent, ou selon le cas CONQ'₁Q'₂, comporte de 6 à 10 groupes OH; on préfère encore que R₂ et R₄ soient identiques et représentent CO-NQ₁Q₂, qui comportent chacun 6 à 10 OH, ou le groupe III dans lequel chaque CONQ'₁Q'₂ comporte de 6 à 10 OH; on préfère aussi les composés dans lesquels R₁, R₃ et R₅ sont choisis parmi les atomes d'iode ou de brome, ainsi que R'₁, R'₃ et R'₅ lorsqu'ils sont présents.

La relaxivité des composés et leur pharmacocinétique in vivo dépendent notamment de leur nombre de noyaux phényles. Par exemple, on peut distinguer les composés dans lesquels p et q sont 0, notamment lorsque R₂ et R₄ représentent CO-NQ₁Q₂ et ceux dans lesquels la somme de p et q vaut de 1 à 3, ou mieux 1 ou 2, et R₂ et R₄ représentent ou non la formule III.

Lorsque dans les composés de formule I, R₂ et R₄ représentent CONQ₁Q₂, Q₁ et Q₂ sont préférablement des groupes alkyles en C₂ à C₆, éventuellement interrompus par un atome d'oxygène.

On préfère d'autre part parmi les composés de formule I ceux dans lesquels Q est H et parmi ceux-ci, ceux dans lesquels Z est CH₂ ou CH₂CONH, Z' est un groupe choisi parmi CONH, CONHCH₂CONH, NHCONH et Z" parmi CONH et CONHCH₂CONH, ainsi que, lorsqu'il est présent, que Z"' soit CONH ou CONHCH₂CONH.

Enfin un autre groupe de composés particuliers est constitué par celui dans lequel p et q étant égaux à 1, Z est CH₂ ou CH₂CONH, Z' et Z" sont choisis parmi CONH et CONHCH₂CONH, et R₂ et R₄ représentent CONQ₁Q₂ avec de préférence R₁, R₃, R₅ choisis parmi Br et I.

D'autres composés préférés sont ceux définis aux points (i) à (ix) ci-dessous :
(i) chelate du composé de formule I dans laquelle p et q sont 0 et R₂ et R₄ identiques représentent -CO-NQ₁Q₂, chaque groupe -CO-NQ₁Q₂ comportant de 6 à 10 groupes -OH;
(ii) chelate du composé de formule I dans laquelle p et q sont 0 ; R₁, R₃ et R₅ sont identiques et choisis parmi Br et I ; et R₂ et R₄, identiques, représentent -CO-NQ₁Q₂, chaque groupe -CO-NQ₁Q₂ comportant de 6 à 10 groupes -OH ;
(iii) chelate du composé de formule I dans laquelle la somme p + q n'est pas 0 ; tout groupe -CO-NQ₁Q₂ présent comporte de 6 à 10 groupes -OH ; R₂ et R₄ ne représentant pas la formule III. Parmi ces composés, ceux pour lesquels p + q est 1 ou 2 sont encore préférés.
(iv) Chelate du composé de formule I, dans laquelle la somme p + q n'est pas 0 ; R₁, R₃ et R₅ sont identiques et choisis parmi Br et I ; et R₂ et R₄, identiques, représentent -CO-NQ₁Q₂, R₂ et R₄ comportant chacun de 6 à 10 groupes -OH. Parmi ces composés, ceux pour lesquels p + q est 1 ou 2 sont encore préférés.
(v) Chelate du composé de formule I dans laquelle Z est CH₂ ou CH₂-CO-NH ; Z' est un groupe choisi parmi CO-NH, CO-NH-CH₂-CO-NH et NH-CO-NH ; Z" est un groupe choisi parmi CO-NH et CO-NH-CH₂-CO-NH ; R₂ et R₄, identiques, représentent -CO-NQ₁Q₂, R₂ et R₄ comportant chacun de 6 à 10 groupes -OH ; et R₁, R₃ et R₅ sont identiques et sont choisis parmi Br et I. Parmi ces composés, ceux pour lesquels p + q est 1 ou 2 sont encore préférés.
(vi) chelate du composé de formule I dans laquelle Z est CH₂ ou CH₂-CO-NH ; Z' est un groupe choisi parmi CO-NH, CO-NH-CH₂-CO-NH, NH-CO-NH ; Z" est choisi parmi CO-NH et CO-NH-CH₂-CO-NH ; Z"' lorsqu'il est présent est CO-NH ou CO-NH-CH₂-CO-NH ; et la somme p + q est 1 ou 2 ;
(vii) chelate du composé de formule I dans laquelle R₂ et R₄ représentent CONQ₁Q₂ et Q₁ et Q₂ sont des groupes alkyle en C₂ à C₆, polyhydroxylés, éventuellement interrompus par un atome d'oxygène ;
(viii) chelate du composé de formule I dans laquelle Z est CH₂ ou CH₂-CO-NH, Z' et Z" sont choisis parmi CO-NH et CO-NH-CH₂-CO-NH, p et q sont égaux à 1 et R₂ et R₄ représentent CONQ₁Q₂. Parmi ces composés, on préfère ceux pour lesquels :
   → R₂ et R₄ comportent ensemble de 6 à 20 OH ; ou
   → R₁, R₃ et R₅ sont identiques et choisis parmi Br et I ; et R₂ et R₄ comportent chacun 6 à 10 groupes OH ;
(ix) chelate du composé de formule I, dans laquelle la somme p + q est 0 ; R₂ et R₄ représentent la formule III et R₁, R₃ et R₅ sont identiques et choisis parmi Br et I.

L'invention concerne aussi un procédé de préparation des composés de formule I qui consiste
- à faire réagir sur le macrocycle condensé de formule 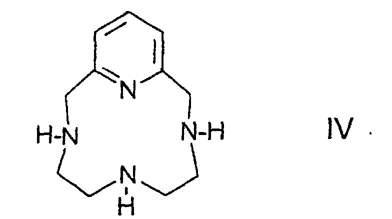 un composé de formule R'OOC-CHX-(CH₂)₂-COOR' dans laquelle X est un groupe partant, tel qu'un atome d'halogène, de préférence le brome, un groupe (C₁-C₃)alkyle sulfonate, tosylate ou triflate et R' est H ou (C₁-C₃)alkyle ou benzyle, et à hydrolyser ou hydrogéner les fonctions esters lorsque R' est différent de H,
   pour obtenir l'hexaacide de formule 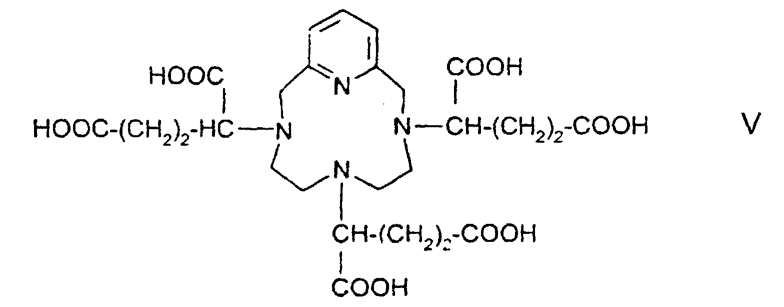
- puis à faire réagir un sel ou un oxyde du métal à complexer, sur l'hexaacide pour obtenir le chelate correspondant ou l'un de ses sels avec une base,
- et enfin à faire réagir sur le chelate, en présence d'un agent activant des fonctions acides carboxyliques, l'amine RNH₂, dans laquelle R a la même signification que dans la formule I, pour obtenir le triamide de formule I.

L'acide de formule V et ses chelates métalliques, notamment celui du gadolinium, et leurs sels, avec une base, telle que NaOH, intermédiaires dans la synthèse des produits de formule I, sont un autre objet de l'invention.

L'invention concerne aussi les compositions contenant un composé de formule I, pour l'imagerie par résonance magnétique lorsque M représente un cation paramagnétique, ou pour la médecine nucléaire lorsque M représente un radioélément ou pour la radiologie lorsque M est le cation d'un atome lourd absorbant les rayons X, les dites compositions pouvant contenir les additifs et véhicules habituels pour une administration par voie orale ou parentérale.

Enfin, l'invention concerne les méthodes d'imagerie médicale qui consistent à administrer au patient une composition contenant un composé de formule I et à observer la région à étudier obtenue par résonance magnétique, par scintigraphie ou sous rayons X.

Les compositions de diagnostic de l'invention peuvent contenir avec un composé de formule I, des additifs tels que antioxydants, tampons, régulateurs de l'osmolalité, stabilisants, sels de calcium, magnésium ou zinc, ou de faibles proportions d'autres chelates de ces cations ou de composés complexants. Des exemples de formulation figurent dans les ouvrages généraux et notamment dans Remington's for Pharmaceutical Science 18e Edition (1990), Mack. Pub. Cy.

Les doses unitaires seront fonction de la nature du produit de contraste, de la voie d'administration, ainsi que du patient et notamment de la nature du trouble à étudier. Pour une injection intraveineuse et une observation par résonance magnétique, la concentration de la solution sera comprise entre 0,001 et 0,5 mole/litre, et on administrera selon le cas de 0,001 à 0,1 millimoles/kilo au patient.

Les produits de contraste de l'invention peuvent être utilisés pour visualiser le cerveau, les organes comme le coeur, le foie ou les reins et tout ou partie du système vasculaire, et pour étudier la perfusion de ces zones et caractériser les anomalies de perméabilité, tumorale, inflammatoire ou ischémique.

Les différentes étapes de la synthèse des composés de l'invention sont réalisés dans des conditions analogues à celles décrites dans la littérature pour des réactions de même type.

Le macrocycle de formule IV peut être préparé par la méthode de Richman et Atkins décrite dans Inorg. Chem. 32, 5257-5265 (1993).

La substitution des atomes d'azote est effectuée, par exemple, par action d'un ester α-bromoglutarique, en présence d'une base minérale ou organique telle que NaOH, Na₂CO₃ ou N(C₂H₅)₃ en solution dans un solvant polaire, tel qu'un alcool ou de préférence aprotique, comme l'acétonitrile ou le tétrahydrofurane.

L'hydrolyse des fonctions esters est obtenue par action d'une base ou d'un acide en milieu aqueux ou hydroalcoolique.

La complexation est réalisée de façon classique, par exemple comme décrit dans US 5,554,748 ou dans Helv. Chim. Acta 69, 2067-2074, (1986).

Pour obtenir le chelate de gadolinium, on peut faire réagir GdCl₃ ou Gd₂O₃ sur le composé de formule V en solution aqueuse à pH compris entre 5 et 6,5. On peut aussi effectuer l'échange du cation d'un complexe de formule V ou I, lorsque la stabilité relative des deux complexes le permet, notamment avec une résine échangeuse d'ions.

Le pourcentage relatif des isomères dans le mélange obtenu, dû à la présence des trois carbone asymétriques, peut être modifié en maintenant quelques jours à une température supérieure à 80° C, une solution aqueuse du chelate de pH voisin de 3.

La réaction d'amidification peut être réalisée en milieu aqueux, éventuellement en présence d'un tiers solvant tel que dioxanne ou tétrahydrofurane, avec un agent activant, comme un carbodiimide soluble tel que ceux porteurs d'un groupe amine décrit dans J. Or. Chem. 21, 439-441, (1956) et 26, 2525-2528, (1961) ou US 3,135,748 ou d'un groupe ammonium quaternaire décrits dans Org. Synth. V, 555-558, qui concerne le 1-éthyl-3-(3-diméthylaminopropyl)carbodiimide (EDCl) et le 1-cyclohexyl 3-(2-morpholinoéthyl)carbodiimide métho p-tolyl sulfonate. Elle peut aussi être effectuée avec le N-hydroxy-sulfosuccinimide comme décrit dans Bioconjugate Chem. 5, 565-576 (1994), ou le 2-succinimido 1,1,3,3-tétra-méthyluronium tétrafluoroborate et analogues, décrits dans Tetrahedron Letters 30, 1927-1930 (1989).

Un autre procédé consiste à former un ester activé intermédiaire en faisant réagir par exemple le N-hydroxysulfosuccinimide (NHS) ou l'hydroxybenzotriazole (HOBT) en présence de carbodiimide, tel que l'EDCI, sur le chelate V qui peut être solubilisé par salification avec un cation minéral, par exemple un ammonium ou sodium.

Avec la 2-éthoxy 1-éthoxycarbonyl 1,2-dihydroquinoléine (EEDQ), on peut effectuer la réaction en milieu hydroalcoolique.

Certaines des aminés RNH₂ sont des composés connus; les autres seront préparées par des procédés d'analogie, de préférence en fixant les noyaux phényles de proche en proche à partir du phényle comportant les groupes R₁ à R₅ et un substituant convenable pour former selon le cas Z, Z', ou Z".

On peut aussi se référer aux brevets WO 96109281 ou WO 97/01359 pour la préparation de celles dans lesquelles Z est CH₂CONH, p = q = 0, R₁ = R₃ = R₅ = halogène ou H, et R₂ = R₄ = CONQ₁Q₂.

Dans certains des aminoalcools précurseurs HNQ₁Q₂, Q₁ et/ou Q₂ représentent le groupe CH₂(CHOH)ₙ(CH₂OCH₂)ᵣ(CHOH)ₜCH₂OH avec t = 0, r = 0,1, n = 0 à 4; ils peuvent être préparés
- à partir d'un aminoalcool ou d'une alkylamine primaire sur lequel on fait réagir un sucre avant d'effectuer la réduction de l'imine obtenue comme décrit dans EP-A-675105 ou EP-A-558395,
- ou à partir de la benzylamine sur laquelle on fait réagir un sucre puis un halogénure ou sulfate d'alkyle en C₁-C₆, éventuellement hydroxylé, avant d'éliminer le groupe benzyle par hydrogénation catalytique.

Suivant la configuration du sucre mis en réaction, on obtiendra des stéréoisomères différents.

Les aminoalcools sont, lorsque r = 1, n = t = 0, préparables à partir du 2-amino-éthoxyéthanol sur lequel on fait réagir un époxyde ou un halogénure d'alkyle hydroxylé convenable, ou encore un aldéhyde aliphatique hydroxylé tel qu'un monosaccharide, pour former une imine, ensuite réduite par voie catalytique ou chimique.

Lorsque r = n = 1 on peut préparer les aminoalcools par action de l'époxyde 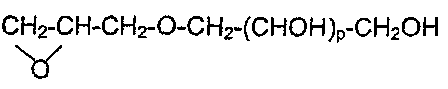 sur l'aminoalcool primaire convenable, lesdits époxydes étant obtenus par oxydation des dérivés éthyléniques correspondants, par une peracide ou un acide peroxyimidique, comme décrit dans J. Org. Chem. 26, 659-663, (1961) et 48, 888-890, (1983).

Parmi les aminoalcools préparés par ces méthodes, on peut citer ceux pour lesquels
Q₁ = CH₂(CHOH)₄CH₂OH et Q₂ = Q₁ ou CH₂(CHOH)CH₂OH
Q₁ = CH₂(CHOH)₂CH₂OH et Q₂ = CH₂CHOHCH₂O(CH₂)₂OH ou
(CH₂)₂OCH₂CHOHCH₂OH
Q₁ = CH₂(CHOH)₃CH₂OH et Q₂ = (CH₂)₂O(CH₂)₂OH.

Dans le cas où p et/ou q sont différents de zéro, on formera le pont Z" entre les deux noyaux phényles, selon le cas, avant ou après le pont Z'.

Par exemple, le composé 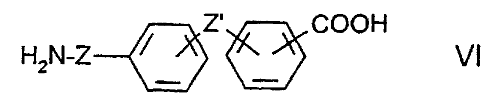 peut être préparé, quand Z est une liaison, à partir des dérivés diphényles VII ou leurs esters 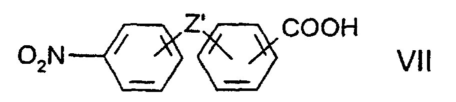 dans lesquels Z' a la même signification que dans la formule II.

Le composé VII dans lequel Z' est O est décrit dans Makromoleculare Chemie 130, 103-144 (1969), celui dans lequel Z' est NH est décrit dans Indian J. Chem. 13, 35-37 (1975), celui dans lequel Z' est CH₂ ou CO dans J. Pharm. Sci. 55(3), 295-302 (1966), celui dans lequel Z' est une liaison dans Synth. Comm. 24(22) 3307-3313 (1994), et celui dans lequel Z' est S est décrit dans II Farmaco, 44(7-8), 683-684 (1989).

D'autres composés VII peuvent être préparés par des procédés d'analogie; par exemple, lorsque Z' est HNCONH, par action de O₂NC₆H₄NCO sur H₂NC₆H₄COOH en milieu anhydre, ou lorsque Z' est NHCO ou CONH, par réaction du chlorure d'acide aromatique sur l'aniline convenable en solution dans un solvant aprotique tel que CH₂Cl₂, C₆H₅CH₃, CH₃CON(CH₃)₂, ou par réaction de l'acide aromatique sur l'aniline en présence de chlorure d'acide sulfonique, de triéthylamine et de diméthylaminopyridine comme décrit dans Synth. Communications 25(18), 2877-2881 (1995).

La réduction du groupe NO₂ de VII en NH₂ peut être effectuée, de manière connue, par l'hydrogène en présence de catalyseur, ou par voie chimique.

Lorsque Z dans la formule VI est CH₂-CONH, on fait réagir la glycine dont la fonction acide est activée et le groupe NH₂ est protégé, sur un composé VI dans lequel Z est une liaison ou sur l'aniline portant un groupe précurseur de Z', éventuellement protégé.

La glycine est protégée, par exemple, sous forme de carbamate, en particulier t-butylcarbamate (Synthesis, 48, (1986)) et benzylcarbamate (Chem. Ber., 65, 1192 (1932)), sous forme de phtalimide (Tetrahedron Letters 25, 20, 2093-2096 (1984)), avec un benzyl (Bull. Soc. Chim. Fr., 1012-1015, (1954)), un N-allyl (Tetrahedron Letters 22, 16, 1483-1486 (1981)). (Voir aussi Protective Groups in Organic Synthesis, 315-349, T.W. Greene (John Wiley & Sons Inc.). L'élimination du groupe protecteur du NH₂ fixé sur Z n'est, en général, effectuée qu'après la constitution du groupe R; de façon classique, un groupe phtalimido est éliminé par action de l'hydrazine, alors qu'un groupe benzyloxycarbonyle ou benzyle l'est par hydrogénation catalytique.

Lorsque Z = CH₂ et Z' = CONH ou CONHCH₂CONH, on peut faire réagir l'acide 4-aminométhylbenzoïque dans lequel le groupe NH₂ est protégé, sous forme de carbamate ou d'imide comme décrit dans J. Org. Chem. 43, 2320-2325 (1978) ou dans Rec. Trav. Chim. Pays-Bas, 79, 688 (1960), sur l'acide benzoïque substitué dont la fonction acide est bloquée par estérification.

Lorsque Z est (CH₂)₂NHCO, on peut préparer RNH₂ par action d'un excès d'éthylènediamine sur un ester benzoïque convenable, portant un groupe précurseur de Z', éventuellement protégé, ou une fraction plus complète de R.

Le composé VI après protection du groupe NH₂, activé sous forme de chlorure d'acide ou par un agent de couplage peptidique, réagira sur le groupe précurseur de Z" porté par le noyau phényle terminal, convenablement substitué par R₁ à R₅ pour donner RNH₂ après déprotection.

Les conditions de préparation des amines RNH₂ seront mieux comprises à la lecture des exemples qui suivent. Les spectres de masse de ces produits (electrospray) comme ceux des exemples correspondent aux structures attendues.

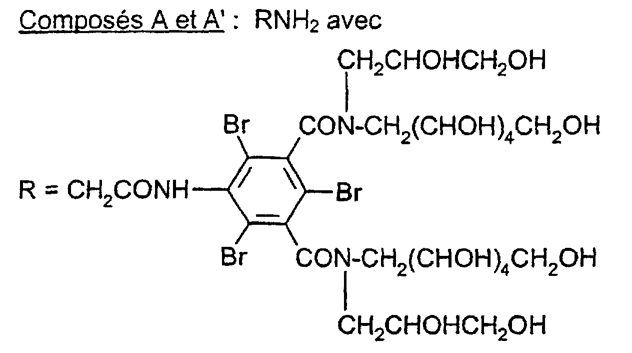

### Composé A :

(a) 1-déoxy 1-(2,3-dihydroxypropyl)amino D-galactitol à partir de α-D-galactose:
   On dissout 35 g de D-galactose dans 100 ml de méthanol contenant 17 g de 3-aminopropanediol et le mélange est maintenu sous agitation à 25°C pendant 12 heures, avant d'introduire 5 g de catalyseur charbon palladié à 10% et 40 ml d'eau pour effectuer l'hydrogénation de l'imine à 60°C. On élimine le catalyseur par filtration et on concentre le milieu jusqu'à 85 ml. L'aminoalcool est isolé par précipitation lors de l'introduction de la solution concentrée dans 30 ml d'isopropanol vers 35°C.
(b) Acide 5-amino 2,4,6-tribromo isophtalique:
   On introduit lentement 156 g de brome dans 300 ml d'une solution aqueuse de 50 g d'acide 5-aminoisophtalique et 55 ml d'acide chlorhydrique à 37%. Après une nuit d'agitation on neutralise l'excès de brome par addition d'une solution aqueuse de bisulfite de sodium avant d'isoler le précipité. Rendement: 90%.
(c) Acide 5-(phtalimidoacétamido) 2,4,6-tribromo isophtalique:
   On introduit lentement 27 ml de chlorure de thionyle dans une solution de 69 g de N-phtaloylglycine, dans 200 ml de diméthylacétamide à 10°C, puis après 2 heures d'agitation on introduit vers 15-20°C, 100 g de l'acide obtenu précédemment Après une nuit à température ambiante, le mélange est versé dans 800 ml d'eau chaude. On isole ainsi 140 g de produit final.
(d) Chlorure du diacide précédent:
   On introduit lentement à 18°C dans une solution de 100 g du diacide dans 300 ml de dioxanne et 50 ml de diméthylformamide, 70 ml de chlorure de thionyle. Le précipité jaune formé après 3 journées d'agitation à température ambiante est filtré et lavé avec de l'oxyde de méthyle et de t-butyle. On obtient ainsi 70 g de solide beige.
(e) N',N'-bls(2,3,4,5,6-pentahydroxyhexyl)N,N'-bis(2,3-dihydroxypropyl) 2,4,6-tribromo 5-(phtalimidoacétamido)isophtalamide:
   On dissout 125 g de l'amine dérivée du galactitol obtenue en (a) dans 610 ml de N-méthylpyrrolidone à 80°C avant d'introduire à 60°C 17 g de Na₂CO₃ et 102 g du chlorure de diacide. Après deux heures à cette température, le milieu réactionnel est ramené à température ambiante puis filtré. Le filtrat est introduit dans 1,5 litre d'isopropanol; le précipité formé est dissous dans l'eau et chromatographié sur une résine échangeuse d'ions sous forme H⁺ pour éliminer l'amine de départ. On isole ainsi 136 g de produit solide.
(f) Composé A :
   125 g du phtalimide précédent sont dissous dans 520 ml de N-méthylpyrrolidone et 175 ml d'eau à 70°C et on ajoute 8 ml d'hydrazine, hydrate, avant de maintenir le milieu pendant deux heures à 90°C. Ce milieu est ensuite refroidi jusque vers 20°C puis versé dans 1,6 litre d'éthanol. Le précipité formé est purifié par passage de sa solution aqueuse sur une résine échangeuse d'ions sous forme H⁺.

### Composé A':

(a') En faisant réagir le 3-aminopropanediol sur du D-glucose dans les mêmes conditions que décrites ci-dessus à l'étape (a) pour la préparation du composé A, on obtient le 1-deoxy-1-(2,3-dihydroxypropyl)amino-D-glucitol.
   Le composé A' peut être obtenu par mise en oeuvre des étapes b) à f) décrites ci-dessus pour la préparation du composé A à partir de l'amine dérivée du glucitol obtenue en a') ou bien par mise en oeuvre de l'étape e') suivante.
(e') On dissout 95 g de l'amine dérivée du glucitol obtenue en (a') dans 460 ml de diméthylacétamide à 90° C avant d'introduire à 65° C, 32 ml de triéthylamine et 117 g du chlorure de diacide décrit à l'étape (d) ci-dessus dans la préparation du composé A. Après 4 h 30 à 55-60° C, le milieu réactionnel est ramené à température ambiante et filtré. La solution obtenue, à 50° C, est versée lentement dans une solution aqueuse d'hydrazine (11 ml dans 115 ml d'eau) ; après 3 heures à 80° C, le milieu revenu à température ambiante est acidifié jusqu'à pH 1 par addition d'une solution aqueuse de HCl N. Le précipité est séparé et le filtrat versé dans 3 litres d'éthanol, sous agitation. Le précipité formé est séché puis purifié par diafiltration pour éliminer la majeure partie des molécules de faible masse et la solution obtenue est chromatographiée sur résines échangeuses d'anions et de cations. Rendement 60%.

L'amine finale peut être lyophilisée.

Analyse HPLC sur colonne 2 avec éluant 2 : CF₃COOH dans l'eau pH 3,3/CH₃CN 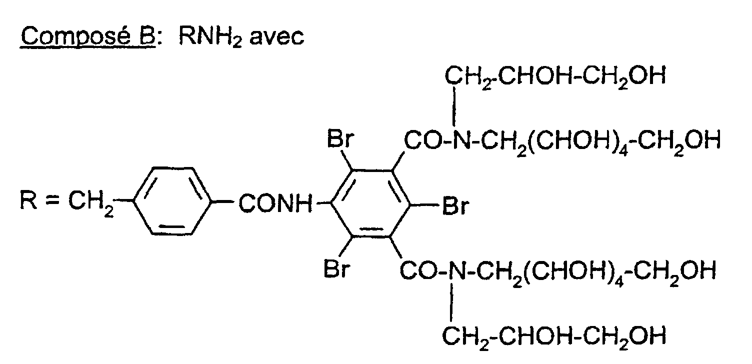
(a) Acide 4-phtalimidométhylbenzoïque:
   On maintient 72 heures à température de reflux un mélange de 10 g d'acide 4-aminométhylbenzoïque, 14,5 g de carbéthoxyphtalimide, 9,2 ml de triéthylamine et 140 ml de tétrahydrofurane. Le précipité formé est isolé à température ambiante; après lavage par une solution aqueuse acide et séchage, on obtient 14,5 g de produit. F = 264°C.
(b) Chlorure de l'acide précédent:
   On introduit dans une solution de 13,5 g de l'acide dans 55 ml de dioxanne, 1 g de chlorure de tricaprylylméthylammonium (aliquat® 336) et 5,3 ml de chlorure de thionyle. Après 12 heures d'agitation à 80°C, le milieu est concentré à sec et le solide résiduel lavé par l'éther diisopropylique. p = 14 g.
(c) Acide 2,4,6-tribromo 5-(phtalimidométhylbenzamido)isophtalique:
   14 g du chlorure d'acide et 15 g d'acide 5-amino 2,4,6-tribromo isophtalique sont dissous dans 50 ml de N-méthylpyrrolidone et le milieu est maintenu à 100°C pendant plusieurs heures. La solution vers 20°C est versée dans 300 ml d'eau et le précipité formé recristallisé dans l'isopropanol pour donner 5,5 g de produit final.
   HPLC (chromatographie liquide haute performance): Colonne No. 1: Symmetry® C18; 100 Å; 5 µm; l = 25 cm; d = 4,6 mm; (Waters).
   Eluant No. 1: CH₃COONH₄ dans l'eau (0,005 M)/CH₃CN.
   Gradient: 80% à 20% (V/V) en 15 minutes; Débit 1 ml/minute;
   tᵣ = 4,5 minutes.
(d) Chlorure de l'acide précédent:
   On introduit dans une solution de 5,5 g de l'acide dans 40 ml de dioxanne, 5,6 ml de diméthylformamide et 9 ml de chlorure de thionyle en maintenant la température à moins de 5°C. Après 30 minutes, le milieu est versé dans 150 ml d'eau et le précipité formé est isolé, p = 4,6 g.
(e) Composé B:
   2,3 g du chlorure d'acide sont introduits dans une solution de 4 g de l'aminoalcool 1-déoxy-1-(2,3-dihydroxypropyl)amino-D-galactitol dans 15 ml de N-méthylpyrrolidone à 65°C. Après 3 heures 30, on ajoute 4 ml d'eau et on porte le milieu à 90°C avant d'ajouter 0,3 ml d'hydrate d'hydrazine. Après 2 heures à 90°C, la solution est versée, à température ambiante, dans 80 ml d'éthanol. Le précipité isolé est dissous dans 10 ml d'eau et la solution à pH 1 est purifiée par chromatographie sur résine anionique sous forme OH⁻, du type Amberlite® puis sur résine cationique sous forme H⁺, de type IMAC®, et enfin sur une résine anionique OH⁻. On obtient 2 g d'amine.
   HPLC: Colonne No. 2: LiChrospher®; 100 RP18; 5 µm; l = 25 cm; d = 4 mm; (Merck®).
   Eluant No. 2: CF₃COOH dans l'eau pH 3,3/CH₃CN.
   Gradient: 98% à 77% (V/V) en 25 minutes; Débit 1 ml/minute;
   tᵣ: 18 à 22 minutes.

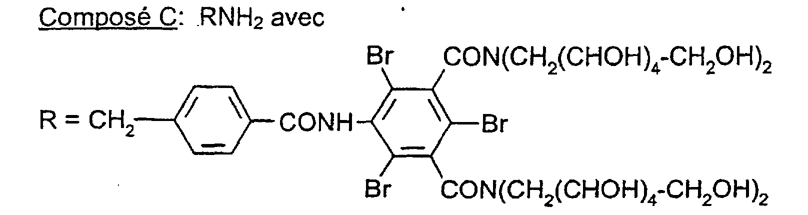

On introduit dans une solution à 65°C de 5,5 g de disorbitylamine dans 30 ml de N-méthylpyrrolidone, 2,3 g du chlorure d'acide préparé selon l'étape (d) précédente. Après 4 heures à 65°C, on introduit 8 ml d'eau puis à 90°C, 0,3 ml d'hydrate d'hydrazine; après 2 heures à cette température, le milieu réactionnel, vers 20°C, est versé sur 130 ml d'eau. Le précipité formé est lavé à l'éthanol puis redissous dans 10 ml d'eau, la solution est amenée à pH 1,5 puis purifiée par chromatographie sur résines anioniques et cationiques. On obtient ainsi 1,7 g de l'amine.
HPLC: Colonne No. 2; Eluant No. 2;
tᵣ: 18 minutes. 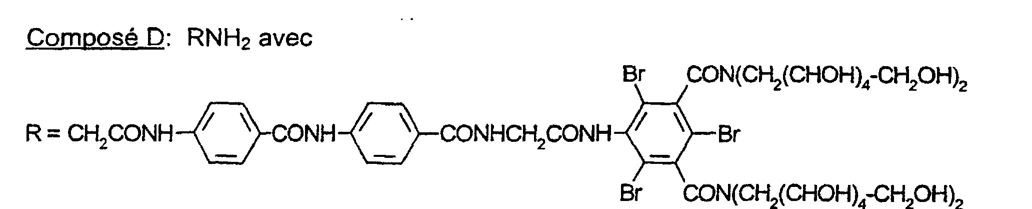
(a) N,N'[-bis(2,3,4,5,6-pentahydroxyhexyl)] 2,4,6-tribromo 5-(glycylamino)-isophtalamide:
   1. On dissout 15 g de disorbitylamine dans 60 ml de N-méthylpyrrolidone à 80°C et on introduit dans le milieu à 60°C, 1,6 g de carbonate de sodium sec, puis 9,6 g du chlorure de l'acide 5-(phtalimidoacétamido) 2,4,6-tribromoisophtalique. Après 1 heure d'agitation à cette température et 16 heures à température ambiante, on élimine le précipité et on verse la solution dans 160 ml d'isopropanol. Le précipité isolé pèse 20 g.
   2. Hydrazinolyse:
      20 g du produit précédent et 1,7 ml d'hydrate d'hydrazine sont introduits dans 40 ml d'eau à 70°C. Après 3 heures d'agitation on acidifie jusqu'à pH 4 par addition d'acide chlorhydrique 6N à température ambiante. On élimine alors le précipité formé et on neutralise le filtrat par addition d'une solution aqueuse de NaOH 1 N. L'hydrazine en excès est éliminée par osmose inverse. La solution résiduelle est traitée par 1 ml de résine cationique forte puis 6,5 ml de résine anionique faible.
      Le produit final est alors extrait de la solution par fixation sur une résine cationique forte sous forme H⁺, d'où elle est éluée par une solution aqueuse diluée de NaCl (0,1 M). p = 8 g.
      HPLC: Colonne No. 2; Eluant No. 3;
      Gradient: CF₃ COOH dans l'eau (pH 3.4)/CH₃CN de 95% à 50% (V/V) en 50 minutes; débit 1 ml/minute;
      tᵣ: 7 minutes.
(b) Acide 4-[4-nitrobenzamido]benzoïque:
   Peu à peu, on introduit 10 g de chlorure d'acide 4-nitrobenzoïque dans 7,4 g d'acide 4-aminobenzoïque et 36 ml de diméthylacétamide, en maintenant la température à moins de 25°C. Après 24 heures d'agitation, on ajoute à 10°C, 50 ml de chlorure de méthylène pour précipiter le produit cherché. Après lavage à l'eau et séchage, on isole 14,5 g de produit.
(c) Acide 4-[4-aminobenzamido]benzoïque:
   On soumet une suspension de 13,6 g de l'acide précédent dans 180 ml d'eau à laquelle on a ajouté 24 ml de solution aqueuse de NaOH 1 N et 1,4 g de palladium sur charbon (10%) à une pression d'hydrogène de 0,6 MPa pendant 4 heures.
   Le pH de la suspension finale est alors amené vers 10 avant filtration sur Celite® pour éliminer le catalyseur. Le précipité formé lors de l'acidification du filtrat jusqu'à pH 5,3 est isolé et séché.
   p = 10,6 g; F > 260°C.
(d) Acide 4-[4-(phtalimidoacétamido)benzamido]benzoïque:
   On introduit dans une solution de 9 g d'acide phtalimidoacétique dans 40 ml de diméthylacétamide à 10°C, 3,2 ml de chlorure de thionyle goutte à goutte puis, après 3 heures d'agitation, 10,5 g de l'aminoacide précédemment obtenu à une température inférieure à 20°C.
   Après 12 heures d'agitation, le milieu est versé dans 400 ml d'eau et le précipité isolé lavé à l'eau chaude. Poids après séchage: 18 g. F > 260°C.
(e) Chlorure de l'acide précèdent:
   On introduit 2,5 ml de chlorure de thionyle dans 10 g de l'acide en suspension dans 50 ml de dioxane, et 1 ml de diméthylformamide et on maintient le mélange sous agitation à 50°C pendant 5 heures. Après addition d'un volume d'éther diisopropylique, on isole 10 g de précipité.
   On peut aussi mettre l'acide en suspension dans le toluène avec du chlorure de tricaprylylméthylammonium comme catalyseur.
(f) N,N'-bis(2,3,4,5,6-pentahydroxyhexyl)2,4,6-tribromo 5-(4[4-(phtatimido acétamido)benzamido]benzoylglycylamino)isophtalamide:
   Une solution de 2,25 g de chlorure d'acide avec 5 g de N,N'-bis(2,3,4,5,6-pentahydroxyhexyl) 2,4,6-tribromo 5-(glycylamino)isophtalamide et 0,7 ml de triéthylamine dans 25 ml de diméthylacétamide ou de N-méthylpyrrolidone est maintenue 12 heures sous agitation puis versée dans 60 ml d'éthanol. On isole ainsi 6,2 g de précipité.
   HPLC: Colonne No. 2; Eluant No. 3;
   tᵣ = 27-35 minutes (mélange d'isomères).
(g) Hydrazinolyse:
   On introduit une solution de 0,6 ml d'hydrate d'hydrazine dans 10 ml d'eau dans une solution de 10 g du phtalimide précédent dans 40 ml de diméthylacétamide à 80°C. Après 3 heures d'agitation à cette température, le mélange refroidi est versé dans 125 ml d'éthanol. On isole 9 g de précipité qui est purifié par traitement de sa solution aqueuse par une résine anionique forte (OH⁻) puis cationique faible (H⁺).
   p = 8 g.
   On peut aussi acidifier le milieu réactionnel pour séparer le phtalylhydrazide précipité et éliminer le solvant et les molécules de faible masse par ultrafiltration avant une précipitation finale dans l'éthanol aqueux.
   HPLC: Colonne No. 2; Eluant No. 3 mais 90/10 (V/V) en élution isochratique à 1 ml/minute;
   tᵣ = 28-35 minutes.

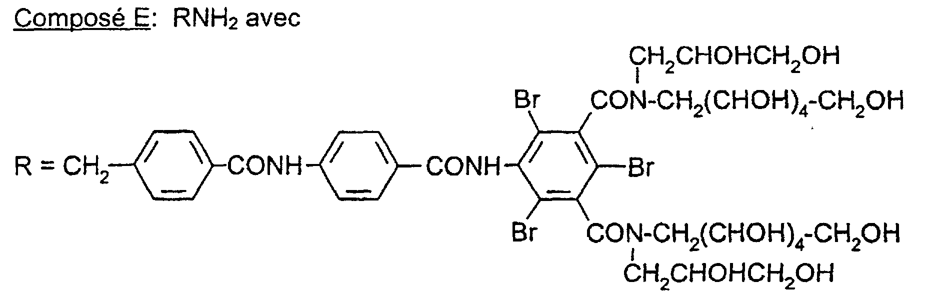

(a) Acide 5-(4-nitrobenzamido) 2,4,6-tribromo isophtalique:
   On maintient pendant 18 heures à la température de reflux, 50 g de chlorure d'acide para-nitrobenzoïque et 75 g d'acide 5-amino 2,4,6-tribromo isophtalique dans 400 ml de dioxanne. Après refroidissement, le précipité est filtré, lavé par 50 ml de dioxanne et séché. p = 115 g.
(b) Acide 5-(4-aminobenzamido) 2,4,6-tribromo isophtalique:
   Une solution de 180 g du dérivé nitré précédent dans 600 ml d'eau est amenée à pH 6 par addition d'une solution aqueuse de NaOH 5N et hydrogénée sous une pression de 5x10⁵ Pa en présence de Pt type 156 (Johnson Matthey) pendant 7 heures. Le catalyseur est séparé par filtration et l'eau évaporée sous pression réduite. p = 80 g.
   HPLC: Colonne No. 2; Eluant No. 4: CF₃ COOH dans l'eau (pH 2,8) avec méthanol (99/1 - VN); débit 1 ml/minute;
   tᵣ: 3,6 minutes (18,8 minutes pour le nitré).
(c) Acide 5-(4-[4-(phtalimidométhyl)benzamido]benzamido) 2,4,6-tribromo isophtalique:
   Un mélange de 10 g d'acide 4-aminométhylbenzoïque, 14,5 g de N-carbéthoxyphtalimide et 9,2 ml de triéthylamine dans 140 ml de tétrahydrofuranne est maintenu à sa température de reflux pendant 72 heures. Le précipité isolé par filtration, à température ambiante, du milieu réactionnel est lavé avec du diéthyléther et une solution aqueuse d'acide chlorhydrique 1 N. On obtient 14,5 g de solide, dont 12,2 g sont dissous à 10°C dans 90 ml de N,N-diméthylacétamide et 3,5 ml de chlorure de thionyle; après 3 heures d'agitation, on introduit dans le milieu 23,4 g de l'aniline obtenue à l'étape précédente et on laisse sous agitation pendant 1 nuit avant de verser le mélange dans 900 ml d'eau. Le précipité isolé, lavé à l'eau est recristallisé dans 200 ml de dioxanne. p = 30 g.
   HPLC: Colonne No. 2; Eluant CF₃COOH dans H₂O (0,1 M)/CH₃CN (90/10 - VN) avec un gradient après 20 minutes jusqu'à 40/60 en 30 minutes; débit 1 ml/minute;
   tᵣ = 26 à 29 minutes.
(d) Dichlorure d'acide:
   30,3 g du dérivé isophtalique obtenu à l'étape précédente sont dissous dans 150 ml de dioxanne contenant 26 ml de diméthylformamide et à 5°C, 42 ml de chlorure de thionyle sont introduits goutte à goutte. Après 30 minutes à O°C, le mélange est versé dans 550 ml d'eau et le précipité formé est filtré, lavé à l'eau et à l'éther diisopropylique. p = 26 g après séchage.
(e) N,N'-bis(2,3,4,5,6-pentahydroxyhexyl)N,N'-bis(2,3-dihydroxypropyl) 2,4,6-tribromo 5-[4-(4-aminomethyibenzamido)benzamido]isophtalamide:
   1. 10 g du dichlorure d'acide sont introduits dans une solution de 15 g de 1-déoxy-1-(2,3-dihydroxypropyl)amino-D-galactitol dans 100 ml de N-méthyl-pyrrolidone à 60°C. Après 4 heures d'agitation à cette température, le milieu, revenu à température ambiante, est versé dans 1 litre d'isopropanol. Le précipité formé est isolé et séché.
      HPLC: Colonne No. 2; Eluant No. 5: CH₃COONH₄ dans H₂O (0,01 M)/CH₃CN; gradient 85% à 50% (V/V) en 20 minutes; débit 1 ml/minute;
      tᵣ = 16 minutes.
   2. Elimination du groupe phtalimide:
      20,4 g du solide précédent sont introduits sous agitation dans 80 ml de N,N-diméthylacétamide à 80°C suivis par 1,6 ml d'hydrate d'hydrazine en solution dans 20 ml d'eau. Après 3 heures à cette température, le milieu réactionnel est versé à température ambiante dans 1 litre d'éthanol. Le précipité formé est isolé, séché puis dissous dans 40 ml d'eau. A 0°C on introduit environ 2 ml de solution aqueuse de HCl 6N pour abaisser le pH à 2; le milieu est filtré sur Célite® puis purifié par passage sur des résines échangeuses d'ions (Amberlite® anionique et IMAC® cationique). On obtient alors 6 g du produit cherché.
      HPLC: Colonne No. 2; Eluant No. 5
      tᵣ = 24 à 29 minutes.

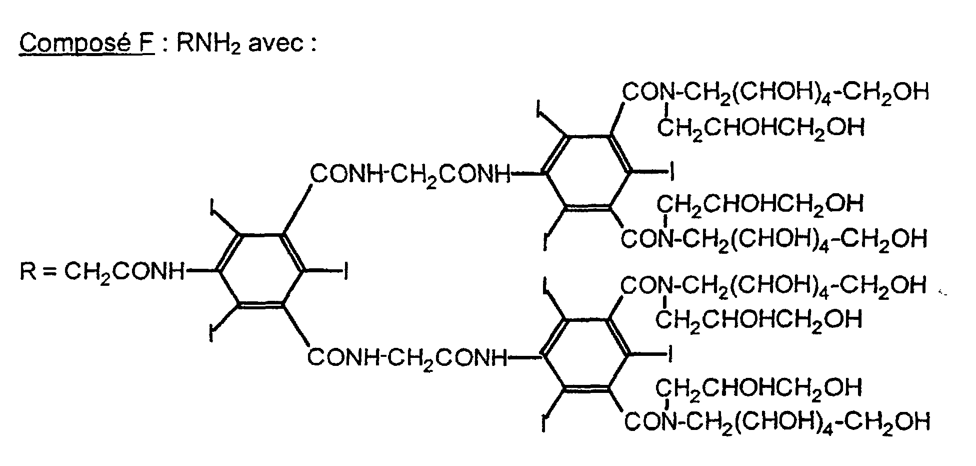

(a) On dissout 50 g de 1-déoxy-1-(2,3-dihydroxypropyl)amino-D-galactitol dans 300 ml de diméthylacétamide à 120° C, puis on ajoute à 80° C rapidement 38 g de chlorure d'acide 5-(phtalimidoacétamido)-2,4,6-triiodoisophtalique (préparé selon US 4,283,381) et 17 ml de triéthylamine. Après 5 heures sous agitation à 80° C, le milieu est filtré à température ambiante et le filtrat introduit dans 800 ml d'alcool isopropylique et le précipité isolé et séché.
   L'excès d'aminoalcool de départ est éliminé par chromatographie sur résine H⁺ de la solution aqueuse de ce précipité.
   On effectue l'hydrazinolyse du groupe phtalimide en milieu aqueux pour obtenir le N,N'-[(2,3,4,5,6-pentahydroxyhexyl)(2,3-dihydroxypropyl)]-2,4,6-triiodo-5-glycylamino isophtalimide.
(b) A 70 g de l'amine obtenue en (a) en solution dans 200 ml de diméthylacétamide, on ajoute 23 g de chlorure d'acide 5-(phtalimidoacétamido) 2,4,6-triiodo-isophtalique et 12 ml de triéthylamine. Après 24 heures d'agitation à température ambiante, le milieu réactionnel est versé dans 1500 ml d'alcool isopropylique et le précipité formé est isolé.

Le phtalimide brut ainsi obtenu, en solution dans 160 ml d'eau, à pH 5, est chromatographié sur 650 g de résine Amberlite® XAD 16 en éluant avec un mélange eau/méthanol (65/35 v:v). La déprotection du groupe amino du phtalimide est effectuée par hydrazinolyse : 57 g de phtalimide sont traités par 3 ml de NH₂-NH₂ dans 200 ml d'eau, à 80° C ; après 3 heures, le phtalylhydrazide est précipité à pH 1 et le filtrat évaporé pour donner une huile qui est purifiée par précipitation dans l'éthanol et chromatographiée sur résine échangeuse anionique (Amberlite® faiblement basique) et cationique (IMAC HPIII de Rohm et Haas).

HPLC analytique : colonne n° 2 - éluant n° 8 : H₂O/CH₃CN ; gradient 95% à 80% en 45 minutes.

tᵣ : 33 à 49 minutes.

Dans les mêmes conditions, l'amine obtenue en (a) est définie par tᵣ = 6 à 16 minutes.

On peut préparer un autre mélange d'isomères du composé F en appliquant le mode opératoire ci-dessus au 1-déoxy-1-(2,3-dihydroxypropyl)amino-D-glucitol.

| | |
|---|---|
| HPLC analytique | colonne n° 2 - éluant n° 8 |
| Produit obtenu à l'étape (a) correspondante | tᵣ = 7 à 24 minutes |
| Produit final | tᵣ = 30 à 40 minutes. |

Dans ce qui suit, on décrit la préparation de certains chelates de l'invention, à titre d'illustration.

### Exemple 1.

Acide 3,6,9,15-tétraazabicyclo[9,3,1]pentadeca-1(15),11,13 triene-3,6,9-tri(α-glutarique) (formule V) et complexe de gadolinium:
1. On dissout 20 g de l'hétérocycle 3,6,9,15-tétraazabicyclo [9,3,1]pentadeca-1(15), 11,13-triene dans 570 ml d'acétonitrile et on introduit à 80°C, 34 g de Na₂CO₃ puis peu à peu 77 g d'α-bromoglutarate de méthyle en solution dans 110 ml d'acétonitrile. Après 24 heures à sa température de reflux, le milieu est filtré vers 20°C et le filtrat amené à sec. Le solide résiduel est purifié par chromatographie sur silice en éluant avec un mélange acétate d'éthyle/heptane (6/4 - V/V). p = 25 g.
2. Hydrolyse:
   On maintient 3 jours à 70°C une solution de 5,8 g du produit obtenu à l'étape précédente dans 15 ml de méthanol auquel 42 ml d'une solution aqueuse de NaOH 5N ont été ajoutés. Après addition de 100 ml d'eau, le pH de la solution est amené à 6,5 par addition d'une résine cationique (H⁺) puis, après filtration, la solution est mise au contact d'une résine anionique OH⁻. Le produit est libéré de la résine par un mélange acide acétique/eau (50/50 - V/V).
   HPLC: Colonne No. 2; Eluant No. 6: H₂SO₄ dans H₂O (0,037 N)/CH₃CN;
   Gradient de 100% à 20% (V/V) en 50 minutes; débit 1 ml/minute;
   tᵣ = 12,5 à 13,5 minutes (plusieurs pics).
3. Complexation:
   3 g de l'acide et 1,9 g de GdCl₃ sont dissous dans 54 ml d'eau. On porte le milieu à 55°C et on amène le pH à 5 par addition d'une solution aqueuse de NaOH N. Après 3 heures à 60°C, le milieu est filtré vers 20°C puis versé dans 100 ml d'éthanol. On isole le sel de sodium du complexe avec un rendement de 70%.
   HPLC: Colonne No. 2; Eluant No. 6;
   tᵣ = 15 à 16 minutes (plusieurs pics).

### Exemple 2.

Composé de formule I
avec

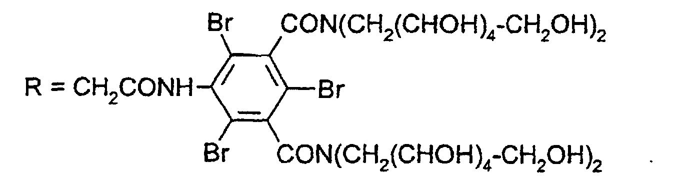

On introduit dans 10 ml d'eau 0,5 g du complexe obtenu à l'exemple 1 et 3,15 g de l'amine RNH₂. Le pH est amené à 6 par addition d'une solution aqueuse de NaOH (O,1N) avant d'ajouter 2 équivalents de 1-(3-diméthylaminopropyl)-3-éthyl-carbodiimide, chlorhydrate (EDCI). Après 3 heures à 40°C, le milieu est versé dans 100 ml d'éthanol et le précipité isolé.

La purification est effectuée par une ultrafiltration de sa solution aqueuse sur une membrane de polyéthersulfone, de seuil de coupure 3 Kdaltons, suivie d'une chromatographie sur colonne de silice silanisée LiChroprep®, (mélange référence RP2 et RP18 (50/50)) de Merck, en éluant avec un mélange eau/CH₃CN (gradient de 98% à 95%). p = 0,8 g.
HPLC: Colonne No. 2; Eluant No. 7: H₂O/CH₃CN;
Gradient de 98% à 70% (V/V) en 20 minutes; débit 1 ml/minute;
tᵣ = 10 minutes.

### Exemple 3.

Composé de formule I
avec

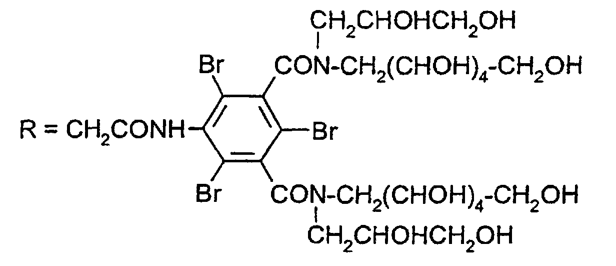

### A) Dérive du galactose :

On dissout 8,3 g du composé A et 1,8 g du complexe obtenu à l'exemple 1 dans 100 ml d'eau. Après avoir amené le pH à 6 par addition d'une solution aqueuse de HCI N, on ajoute 2,7 g d'EDCI et 150 mg de sel de sodium de l'acide (N-hydroxysuccimidyl)-3-sulfonique (NHS). Après 2 heures d'agitation, la solution est versée dans 300 ml d'éthanol et le précipité formé est isolé. Il peut être purifié par chromatographie liquide haute pression préparative sur Lichrospher® C18- 100Å - 12 µm (Merck) ou par ultrafiltration.
HPLC analytique: Colonne No. 2; Eluant No. 7;
tᵣ = 12 minutes.

### A') Dérivé du glucose :

Par réaction du composé A' sur le complexe obtenu à l'exemple 1, dans les mêmes conditions opératoires que pour A, on obtient le composé correspondant de formule I, brut. Celui-ci peut être purifié par chromatographie sur colonne Purospher® RP18, en éluant avec un mélange H₂O/CH₃CN. Rendement final : 40%.

### Exemple 4.

Composé de formule I
avec

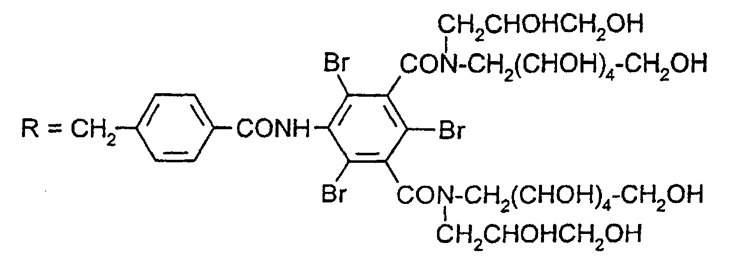

2,5 g du complexe obtenu selon l'exemple 1 et 11 g de composé B sont dissous dans 60 ml d'eau; après addition d'une solution aqueuse de HCI 1N jusqu'à obtention de pH 6, on ajoute 45 ml d'acétone et 0,5 g d'hydroxybenzotriazole, hydrate (HOBT) et 2,3 g d'EDCI. Le milieu réactionnel est agité pendant 4 heures vers 20°C en ramenant de temps en temps le pH à 6 par addition de solution aqueuse de NaOH N. La solution est versée dans 500 ml d'éthanol et le précipité isolé est séché.
p = 11,7 g.

Le produit est purifié par chromatographie préparative sous pression sur une colonne Lichrospher® C18 - 10 µm; I = 25 cm; d = 50 mm (Merck) en éluant avec un mélange eau/acétonitrile avec un gradient de 95% à 90% en 20 minutes puis à 85% en 25 minutes; débit 80 ml/minute. Rendement = 45%.
HPLC analytique: Colonne No. 2; Eluant No. 8: H₂O/CH₃CN; gradient 95% à 80% en 45 minutes;
tᵣ = 31 minutes.

### Exemple 5.

Composé de formule I
avec 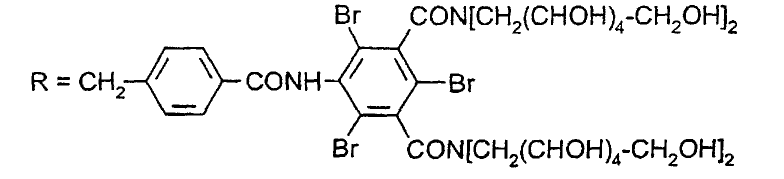 préparé selon la méthode décrite à l'exemple 4 mais avec le composé C.
HPLC analytique: Colonne No. 2; Eluant No 8.
temps de rétention 25 à 29 minutes.

### Exemple 6.

Composé de formule I
avec

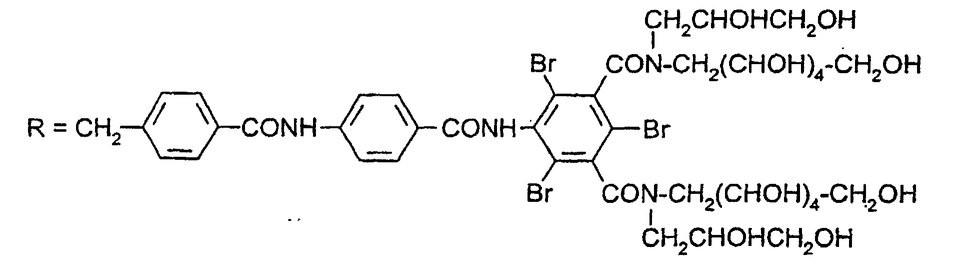

On dissout 1,6 g du complexe de l'exemple 1 et 7 g de l'amine E dans 40 ml d'eau. A pH 6, on introduit 0,3 g d'HOBT et 1,5 g d'EDCI puis 30 ml d'acétone. Le milieu est agité pendant 4 heures en maintenant le pH vers 6. Le précipité obtenu en versant la solution dans 450 ml d'éthanol est purifié par chromatographie préparative haute pression sur une colonne Purospher®; L = 250 mm; d = 40 mm; RP18; 10 µm; 120 Å (Merck) en éluant avec un mélange eau/acétonitrile (gradient 90% à 80% en 5 minutes) ; débit = 80 ml/minute.
HPLC analytique: Colonne No. 3: Purospher®; RP18 endcapped; 5 µm; L = 250 mm; d = 4 mm; (Merck); éluant No. 7 mais gradient de 85% à 70% en 10 minutes (V/V) au bout de 20 minutes;
tᵣ = 29 minutes.

### Exemple 7.

Composé de formule I
avec

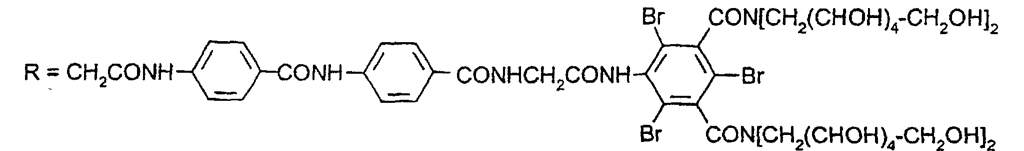

Le produit est préparé en appliquant la méthode décrite dans les exemples précédents avec l'amine D.
HPLC analytique: Colonne No. 4: Zorbax® 3005B - C18; l = 250 mm, d = 4 mm, Hewlett® Packard;
Eluant No. 1 mais gradient 90% à 82% en 60 minutes;
tᵣ = 42 à 49 minutes.

### Exempte 8.

Composé de formule I
avec

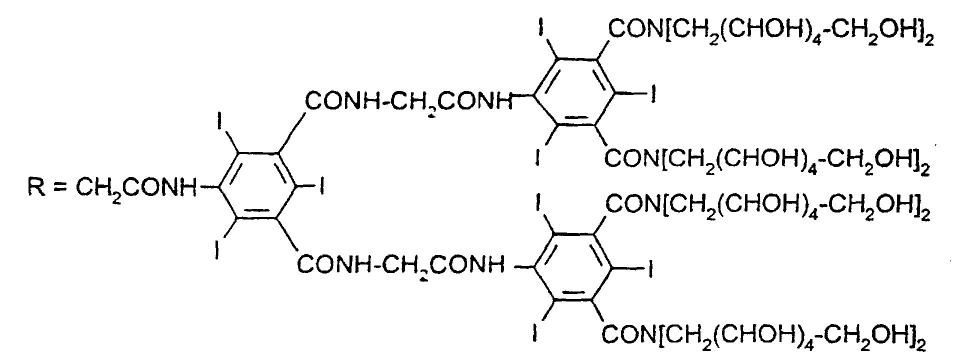

Le produit est préparé en utilisant des conditions d'amidification analogues à celle des exemples précédents.
HPLC analytique: Colonne No. 2
Eluant No. 7 mais gradient 98% à 85% en 40 minutes;
tᵣ = 24 minutes.

### Exemple 9.

Composé de formule I
avec 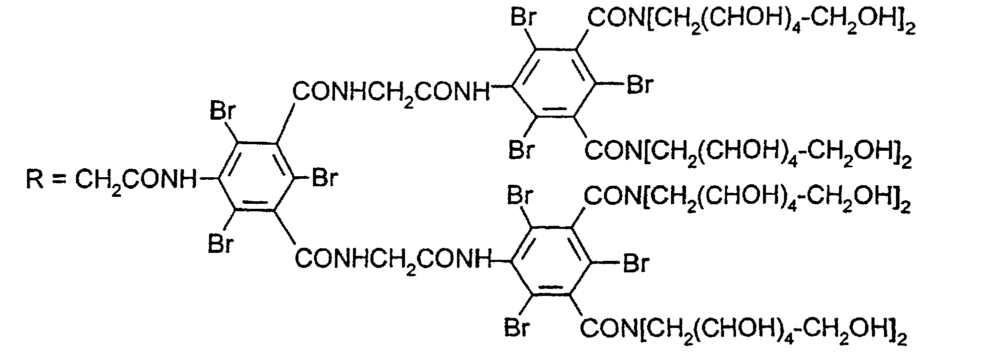 HPLC analytique: Colonne No. 2; Eluant: H₂O/CH₃CN dans 95/5 - V/V puis gradient après 20 minutes pour atteindre 85/15 en 10 minutes; débit = 1 ml/minute.
tᵣ = 26 minutes.

### Exemple 10

Composé de formule I avec 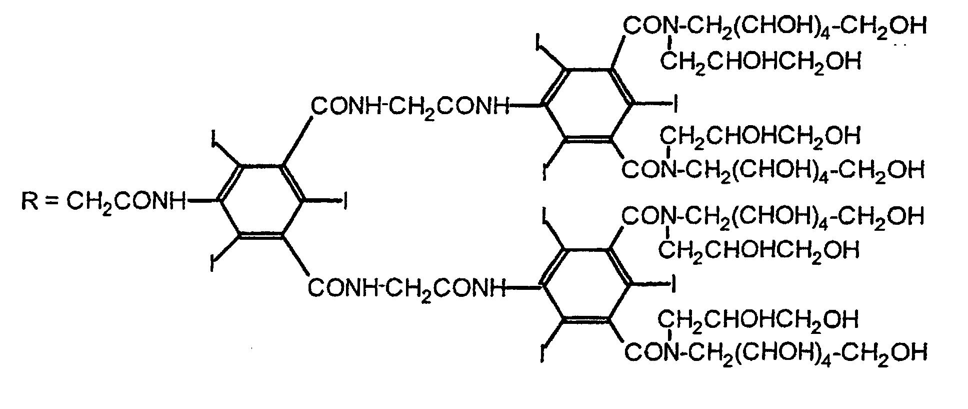 préparé en faisant réagir le composé F avec le complexe de l'exemple 1 :

1 g de complexe et 12 g d'amine F sont dissous dans 200 ml d'eau et le pH est amené à 6 par addition d'une solution aqueuse de HCl 6N. On introduit alors 0,1 g d'EDCI et 0,25 g de HOBT ; le milieu est maintenu sous agitation 48 heures en ramenant le pH à 6 par addition d'une solution aqueuse à 2% de NaHCO₃. Le produit final brut est isolé par introduction du milieu dans 10 volumes d'éthanol et purifié par chromatographie préparative sur colonne Purospher® RP18 10 µm. 120 Å (Merck), en éluant avec un mélange H₂O/CH₃CN. Rendement : 30%.
HPLC analytique : colonne n° 2 - éluant N° 8
tᵣ = 12 minutes.

## Revendications

1. Chelate métallique du composé de formule 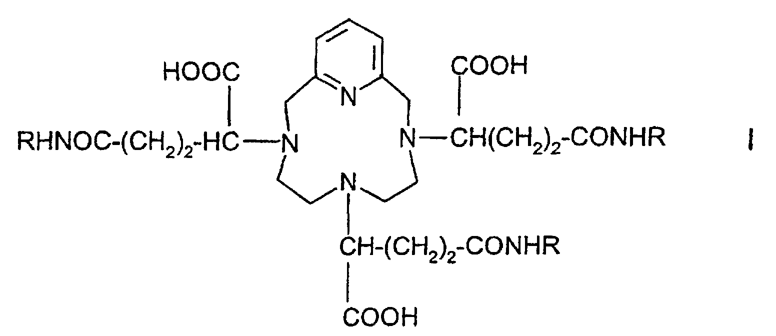 dans laquelle
R représente la formule 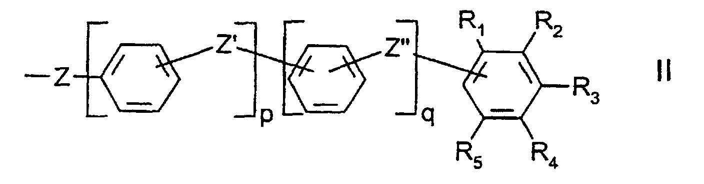 et
- Z est une liaison ou un groupe choisi parmi les groupes CH₂, CH₂-CO-NH ou (CH₂)₂-NH-CO,
- Z' est une liaison ou un groupe choisi parmi les groupes O, S, NQ, CH₂, CO, CO-NQ, NQ-CO, NQ-CO-NQ, CO-NQ-CH₂-CO-NQ,
- Z" est une liaison ou un groupe choisi parmi les groupes CO-NQ, NQ-CO, CO-NQ-CH₂-CO-NQ,
- p et q sont des nombres entiers dont la somme vaut de 0 à 3,
- R₁, R₂, R₃, R₄ et R₅, indépendamment l'un de l'autre, sont choisis parmi les groupes H, Br, Cl, I, CONQ₁Q₂ et NQ₁-CO-Q₂ et Q₁ et Q₂ identiques ou différents étant H ou (C₁-C₆)alkyle éventuellement interrompu par un ou des atomes d'oxygène et au moins l'un des R₁ à R₅ étant un groupe amido
ou R₁, R₃ et R₅ sont indépendamment l'un de l'autre, H, Br, Cl ou I et R₂ et R₄ identiques représentent la formule 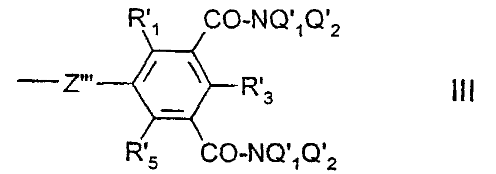 telle que Z"' est un groupe choisi parmi les groupes CO-NQ, CO-NQ-CH₂-CO-NQ, CO-NQ-CH₂, CO-NQ-(CH₂)₂-NQ-CO et NQ-CO-NQ et R'₁, R'₃ et R'₅, identiques ou différents, représentent H, Br, Cl ou I et Q'₁ et Q'₂, identiques ou différents, sont H ou (C₁-C₆)alkyle éventuellement interrompu par un ou des atomes d'oxygène,
- Q représente H ou (C₁-C₄)alkyle,
étant entendu que les groupes alkyles peuvent être éventuellement mono ou polyhydroxylés.

2. Chelate, selon la revendication 1, du composé de formule I, dans laquelle p et q sont 0 et R₂ et R₄ représentent CO-NQ₁Q₂.

3. Chelate, selon la revendication 1, du composé de formule I dans laquelle la somme p + q n'est pas 0 et R₂ et R₄ ne représentent pas la formule III.

4. Chelate, selon la revendication 1, du composé de formule I dans laquelle la somme p + q est 1 ou 2 et R₂ et R₄ ne représentent pas la formule III.

5. Chelate, selon la revendication 1, du composé de formule I dans laquelle les groupes R₁ à R₅ d'un même noyau phényle comportent ensemble de 6 à 20 OH.

6. Chelate, selon l'une des revendications précédentes, du composé de formule I dans laquelle tout groupe CONQ₁Q₂ présent, ou selon le cas CONQ'₁Q'₂ comporte de 6 à 10 groupes OH.

7. Chelate, selon l'une des revendications précédentes, du composé de formule I dans laquelle R₁, R₃ et R₅ sont identiques et choisis parmi Br et et dans laquelle R'₁, R'₃ et R'₅, lorsqu'il sont présents, sont identiques et choisis parmi Br et I.

8. Chelate, selon l'une des revendications précédentes, du composé de formule I dans laquelle Z est CH₂ ou CH₂-CO-NH, Z' est un groupe choisi parmi CO-NH, CO-NH-CH₂-CO-NH, NH-CO-NH et Z" parmi CO-NH et CO-NH-CH₂-CO-NH, et Z"', lorsqu'il est présent, est CO-NH ou CO-NH-CH₂CO-NH.

9. Chelate, selon l'une des revendications précédentes, du composé de formule I dans laquelle R₂ et R₄ représentent CONQ₁Q₂, Q₁ et Q₂ sont des groupes alkyles en C₂ à C₆, éventuellement interrompus par un atome d'oxygène.

10. Chelate, selon l'une des revendications précédentes, du composé de formule I dans laquelle Z est CH₂ ou CH₂-CO-NH, Z' et Z" sont choisis parmi CO-NH et CO-NH-CH₂-CO-NH. p et q sont égaux à 1 et R₂ et R₄ représentent CONQ₁Q₂.

11. Chelate selon la revendication 1 d'un composé de formule I dans laquelle p = q = 0; Z = CH₂CONH; R₁ = R₃ = R₅ = Br;

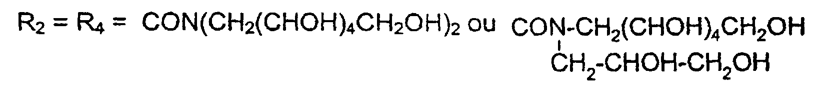

12. Chelate selon la revendication 1 d'un composé de formule I dans laquelle p = 1,2; q = 0; 0; 2 = CH₂; Z' = CONH; R₁ = R₃ = R₅ = Br;

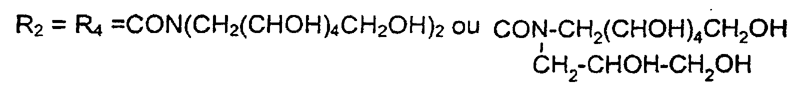

13. Chelate selon la revendication 1 d'un composé de formule I dans laquelle p = q = 1; Z = CH₂CONH; Z' = CONH; Z" = CONH-CH₂CONH; R₁ = R₃ = R₅ = Br; R₂ = R₄ =CON(CH₂(CHOH)₄CH₂OH)₂.

14. Chelate selon la revendication 1 d'un composé de formule I dans laquelle p = q = 0; Z = CH₂CONH; R₁ = R₃ = R₅ = Br; 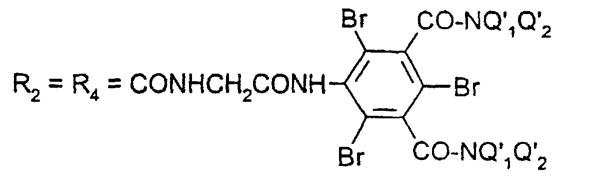 Q'₁ = Q'₂ = CH₂(CHOH)₄CH₂OH
ou Q'₁ = CH₂(CHOH)₄CH₂OH et Q'₂ = CH₂-CHOH-CH₂OH.

15. Chelate selon la revendication 1, du composé de formule I, dans laquelle p et q sont 0, R₂ et R₄ représentent la formule III et R₁, R₃ et R₅ sont identiques et choisis parmi Br et I.

16. Chelate, selon l'une des revendications précédentes, du composé de formule I et de Gd³⁺, ou Mn²⁺.

17. Chelate métallique du composé de formule 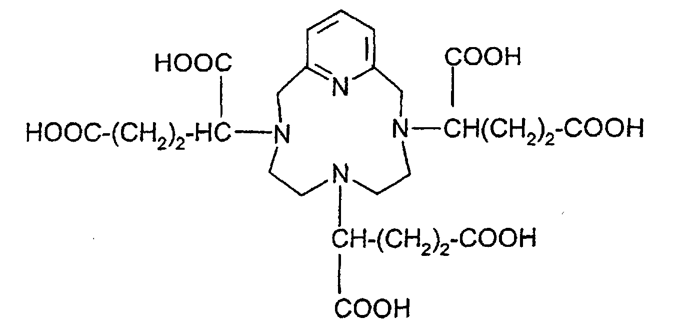 et ses sels avec une base minérale ou organique.

18. Chelate, selon la revendication 17, dans lequel le métal est Gd.

19. Procédé de préparation des composés de formule I, selon la revendication 1, qui consiste
- à faire réagir sur le macrocycle de formule 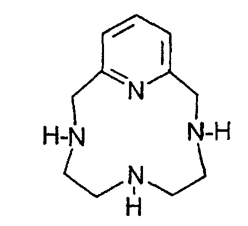 un composé de formule R'OOC-CHX-(CH₂)₂-COOR' dans laquelle X est un groupe partant et R' est H ou (C₁-C₃)alkyle et à hydrolyser les fonctions esters lorsque R' est différent de H,
- puis à faire réagir un sel ou un oxyde métallique pour obtenir le chelate du produit formé ou un de ses sels avec une base,
- et à faire réagir le chelate sur l'amine RNH₂ en présence d'un agent activant les groupes acides carboxyliques.

20. Composition pour l'imagerie diagnostique comprenant un chelate selon l'une des revendications 1 à 16 avec un véhicule pharmaceutiquement acceptable et éventuellement les additifs de formulation habituels.

21. Composition pour l'imagerie par résonance magnétique nucléaire comprenant un chelate de gadolinium selon l'une des revendications 1 à 16.

## Claims

1. Metal chelate of the compound of formula 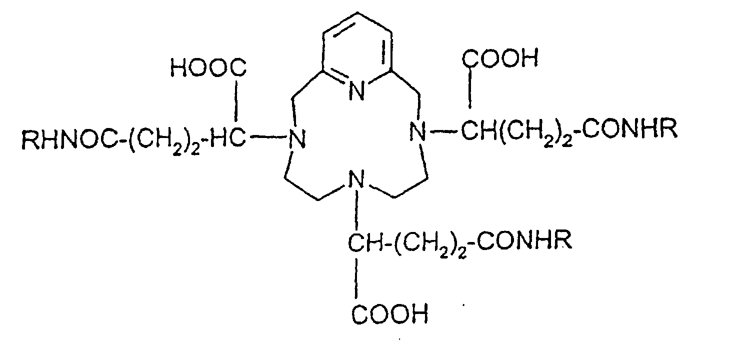 in which R represents the formula 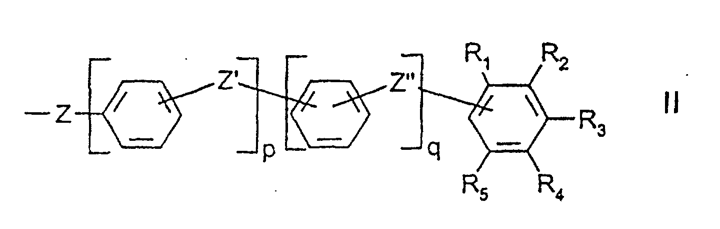 and
- Z is a bond or a group chosen from among the groups CH₂, CH₂-CO-NH or (CH₂)₂-NH-CO,
- Z' is a bond or a group chosen from among the groups O, S, NQ, CH₂, CO, CO-NQ, NQ-CO, NQ-CO-NQ, CO-NQ-CH₂-CO-NQ,
- Z" is a bond or a group chosen from among the groups CO-NQ, NQ-CO, CO-NQ-CH₂-CO-NQ,
- p and q are integers, whose sum is 0 to 3,
- R₁, R₂, R₃, R₄ and R₅, independently of one another, are chosen among the groups H, BR, Cl, I, CONQ₁Q₂ and NQ₁-CO-Q₂ and Q₁ and Q₂, which can be the same or different, are H or (C₁-C₆)alkyl, optionally interrupted by one or more oxygen atoms and at least one of the R₁ to R₅ is an amido group or R₁, R₃ and R₅, independently of one another, are H, Br, Cl or I and R₂ and R₄, which are identical, represent the formula 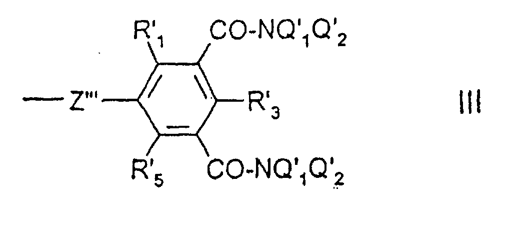 such that Z"' is a group chosen from among the groups CO-NQ, CO-NQ-CH₂-CO-NQ, CO-NQ-CH₂, CO-NQ-(CH₂)₂-NQ-CO and NQ-CO-NQ and R'₁, R'₃ and R'₅, which can be the same or different, represent H, Br, Cl or I and Q'₁ and Q'₂, which can be the same or different, are H or (C₁-C₆)alkyl, optionally interrupted by one or more oxygen atoms,
- Q represents H or (C₁-C₄)alkyl,
it being understood that the alkyl groups can optionally be monohydroxylated or polyhydroxylated.

2. Chelate according to claim 1 of the compound of formula I, in which p and q are 0 and R₂ and R₄ represent CO-NQ₁Q₂.

3. Chelate according to claim 1 of the compound of formula I, in which the sum p + q is not 0 and R₂ and R₄ do not represent the formula III.

4. Chelate according to claim 1 of the compound of formula I, in which the sum p + q is 1 or 2 and R₂ and R₄ do not represent the formula III.

5. Chelate according to claim 1 of the compound of formula I, in which the groups R₁ to R₅ of the same phenyl nucleus have together 6 to 20 OH.

6. Chelate according to one of the preceding claims of the compound of formula I, in which any CONQ₁Q₂ group present or, if appropriate, CONQ'₁Q'₂, has 6 to 10 OH groups.

7. Chelate according to one of the preceding claims of the compound of formula I, in which R₁, R₃ and R₅ are identical and are chosen from among Br and I, and in which R'₁, R'₃ and R'₅, when present, are identical and chosen from among Br and I.

8. Chelate according to one of the preceding claims of the compound of formula I, in which Z is CH₂ or CH₂-CO-NH, Z' is a group chosen from among CO-NH, CO-NH-CH₂-CO-NH, NH-CO-NH and Z" from among CO-NH and CO-NH-CH₂-CO-NH and Z"', when present, is CO-NH or CO-NH-CH₂CO-NH.

9. Chelate according to one of the preceding claims of the compound of formula I, in which R₂ and R₄ represent CONQ₁Q₂, Q₁ and Q₂ are C₂ to C₆ alkyl groups, optionally interrupted by an oxygen atom.

10. Chelate according to one of the preceding claims of the compound of formula I, in which Z is CH₂ or CH₂-CO-NH, Z' and Z" being chosen from among CO-NH and CO-NH-CH₂-CO-NH, p and q being equal to 1 and R₂ and R₄ represent CONQ₁Q₂.

11. Chelate according to claim 1 of a compound of formula I in which p = q = 0; Z = CH₂CONH; R₁ = R₃ = R₅ = Br; R₂ = R₄ = CON(CH₂(CHOH)₄CH₂OH)₂ or

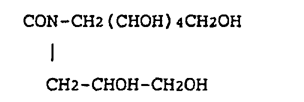

12. Chelate according to claim 1 of a compound of formula I in which p = 1.2; q = 0; Z = CH₂; Z' = CONH; R₁ = R₃ = R₅ = Br; R₂ = R₄ = CON(CH₂(CHOH)₄CH₂OH)₂ or

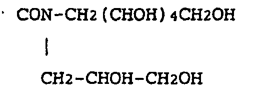

13. Chelate according to claim 1 of a compound of formula I in which p = q = 1; Z = CH₂CONH; Z' = CONH; Z" = CONH-CH₂CONH; R₁ = R₃ = R₅ = Br; R₂ = R₄ = CON(CH₂(CHOH)₄CH₂OH)₂.

14. Chelate according to claim 1 of a compound of formula I, in which p = q = 0; Z = CH₂CONH; R₁ = R₃ = R₅ = Br; 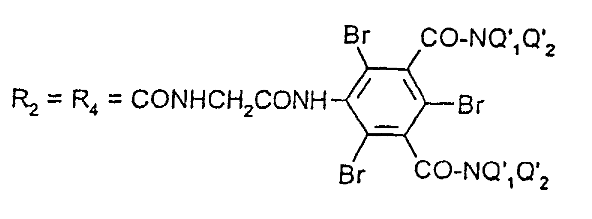 Q'₁ = Q'₂ = CH₂(CHOH)₄CH₂OH
or Q'₁ = CH₂(CHOH)₄CH₂OH and Q'₂ = CH₂-CHOH-CH₂OH.

15. Chelate according to claim 1 of the compound of formula I, in which p and q are 0, R₂ and R₄ represent the formula III and R₁, R₃ and R₅ are identical and are chosen from among Br and I.

16. Chelate according to one of the preceding claims of the compound of formula I and Gd³⁺ or Mn²⁺.

17. Metal chelate of the compound of formula 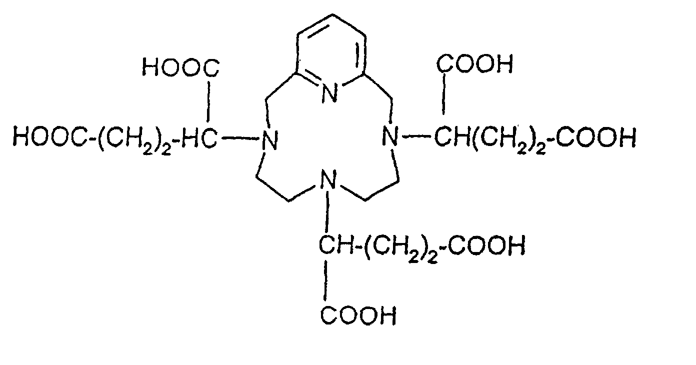 and its salts with a mineral or organic base.

18. Chelate according to claim 17, in which the metal is Gd.

19. Process for the preparation of compounds of formula I, according to claim 1 and which consists of reacting on the macromolecule of formula 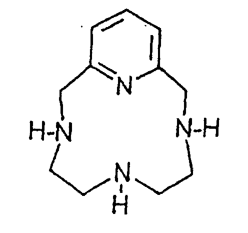 a compound of formula R'OOC-CHX-(CH₂)₂-COOR', in which X is a starting group and R' is H or (C₁-C₃)alkyl and hydrolyzing the ester functions when R' differs from H,
- then reacting a metal oxide or a salt in order to obtain the chelate from the product formed or one of its salts with a base,
- and reacting the chelate on the amine RNH₂ in the presence of an agent activating the carboxylic acid groups.

20. Composition for diagnostic imaging comprising a chelate according to one of the claims 1 to 16 with a pharmaceutically acceptable vehicle and optionally conventional formulation additives.

21. Composition for imaging by nuclear magnetic resonance comprising a gadolinium chelate according to one of the claims 1 to 16.

## Patentansprüche

1. Metallchelat der Verbindung der Formel 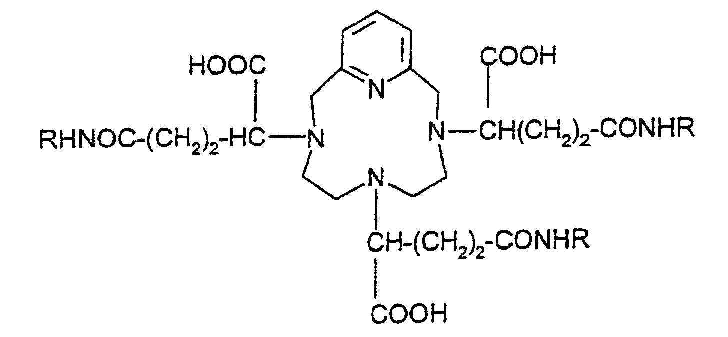 in der
R der Formel entspricht 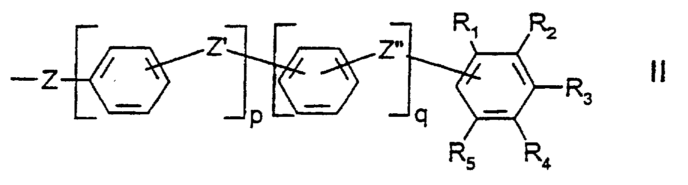 und
- Z eine Bindung oder eine Gruppe ausgewählt aus den Gruppen CH₂, CH₂-CO-NH oder (CH₂)₂-NH-CO darstellt,
- Z' eine Bindung oder eine Gruppe ausgewählt aus den Gruppen O, S, NQ, CH₂, CO, CO-NQ, NQ-CO, NQ-CO-NQ und CO-NQ-CH₂-CO-NQ bedeutet,
- Z" eine Bindung oder eine Gruppe ausgewählt aus den Gruppen CO-NQ, NQ-CO und CO-NQ-CH₂-CO-NQ bedeutet,
- p und q ganze Zahlen darstellen, deren Summe 0 bis 3 beträgt,
- R₁, R₂, R₃, R₄ und R₅ unabhängig voneinander ausgewählt sind aus den Gruppen H, Br, Cl, I, CONQ₁Q₂ und NQ₁-CO-Q₂, worin Q₁ und Q₂ gleichartig oder verschieden sind und H oder (C₁-C₆)-Alkyl, welches gegebenenfalls durch ein oder mehrere Sauerstoffatome unterbrochen ist, darstellen und mindestens einer der Reste R₁ und R₅ eine Amidogruppe bedeutet,
oder R₁, R₃ und R₅ unabhängig voneinander H, Br, Cl oder I bedeuten und R₂ und R₄ identisch sind und der Formel entsprechen: 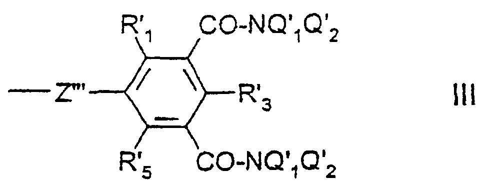 worin Z"' eine Gruppe ist ausgewählt aus den Gruppen CO-NQ, CO-NQ-CH₂-CO-NQ; CO-NQ-CH₂, CO-NQ(CH₂)₂-NQ-CO und -NQ-CO-NQ und R'₁, R'₃ und R'₅, die gleichartig oder verschieden sind, H, Br, Cl oder I bedeuten und Q'₁ und R'₂. die gleichartig oder verschieden sind, H oder (C₁-C₆)-Alkyl, gegebenenfalls unterbrochen durch ein oder mehrere Sauerstoffatome, bedeuten, und
- Q H oder (C₁-C₄)-Alkyl darstellt,
mit der Maßgabe, daß die Alkylgruppen gegebenenfalls mono- oder polyhydroxyliert sein können.

2. Chelat nach Anspruch 1 der Verbindung der Formel I, in der p und q 0 und R₂ und R₄ CO-NQ₁Q₂ bedeuten.

3. Chelat nach Anspruch 1 der Verbindung der Formel I, in der die Summe von p + q nicht 0 beträgt und R₂ und R₄ nicht der Formel III entsprechen.

4. Chelat nach Anspruch 1 der Verbindung der Formel I, in der die Summe von p + q 1 oder 2 beträgt und R₂ und R₄ nicht der Formel III entsprechen.

5. Chelat nach Anspruch 1 der Verbindung der Formel I, in der die Gruppen R₁ bis R₅ ein und desselben Phenylkerns gemeinsam 6 bis 20 OH-Gruppen tragen.

6. Chelat nach einem der vorhergehenden Ansprüche der Verbindung der Formel I, in der jede vorhandene Gruppe CONQ₁Q₂ oder gegebenenfalls CONQ'₁Q'₂ 6 bis 10 OH-Gruppen aufweist.

7. Chelat nach einem der vorhergehenden Ansprüche der Verbindung der Formel I, in der R₁, R₃ und R₅ gleichartig sind und ausgewählt aus Br und I, und worin R'₁, R'₃ und R'₅, falls sie vorhanden sind, gleichartig sind und aus Br und I ausgewählt sind.

8. Chelat nach einem der vorhergehenden Ansprüche der Verbindung der Formel I, in der Z CH₂ oder CH₂-CO-NH, Z' eine Gruppe ausgewählt aus CO-NH. CO-NH-CH₂-CO-NH und NH-CO-NH und Z" ausgewählt aus CO-NH und CO-NH-CH₂-CO-NH und Z"', falls es vorhanden ist, CO-NH oder CO-NH-CH₂-CO-NH bedeuten.

9. Chelat nach einem der vorhergehenden Ansprüche der Verbindung der Formel I, in der R₂ und R₃ CONQ₁Q₂ bedeuten, worin Q₁ und Q₂ C₂-C₆-Alkylgruppen darstellen, die gegebenenfalls durch ein Sauerstoffatom unterbrochen sind.

10. Chelat nach einem der vorhergehenden Ansprüche der Verbindung der Formel I, in der Z CH₂ oder CH₂-CO-NH darstellt, Z' und Z" ausgewählt sind aus CO-NH und CO-NH-CH₂-CO-NH, p und q den Wert 1 besitzen und R₂ und R₄ CONQ₁Q₂ darstellen.

11. Chelat nach Anspruch 1 einer Verbindung der Formel I, in der p = q = 0; Z = CH₂CONH; R₁ = R₃ = R₅ = Br; R₂ = R₄ = CON(CH₂(CHOH)₄CH₂OH)₂ oder 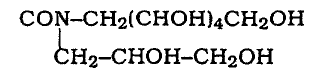 bedeuten.

12. Chelat nach Anspruch 1 einer Verbindung der Formel I, in der p = 1,2; q = 0; Z = CH₂; Z' = CONH; R₁ = R₃ = R₅ = Br; R₂ = R₄ = CON(CH₂(CHOH)₄CH₂OH)₂ oder 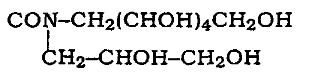 bedeuten.

13. Chelat nach Anspruch 1 einer Verbindung der Formel I, in der p = q = 1; Z = CH₂CONH; Z' = CONH; Z" = CONH-CH₂-CONH; R₁ = R₃ = R₅ = Br: R₂ = R₄ = CON(CH₂(CHOH)₄CH₂OH)₂
bedeuten.

14. Chelat nach Anspruch 1 einer Verbindung der Formel I, in der p = q = 0; Z = CH₂CONH; R₁ = R₃ = R₅ = Br; 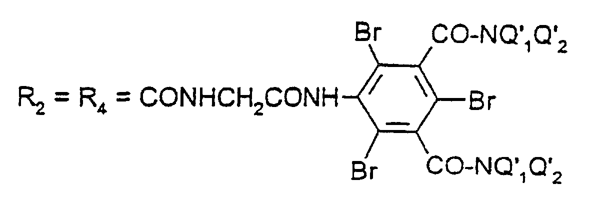 Q'₁ = Q'₂ = CH₂(CHOH)₄CH₂OH oder
Q'₁ = CH₂(CHOH)₄CH₂OH und Q'₂ = CH₂-CHOH-CH₂OH
bedeuten.

15. Chelat nach Anspruch 1 der Verbindung der Formel I, in der p und q 0 bedeuten, R₂ und R₄ der Formel III entsprechen und R₁, R₃ und R₅ identisch sind und ausgewählt aus Br und I.

16. Chelat nach einem der vorhergehenden Ansprüche der Verbindung der Formel I und Gd³⁺ oder Mn²⁺.

17. Metallchelat der Verbindung der Formel 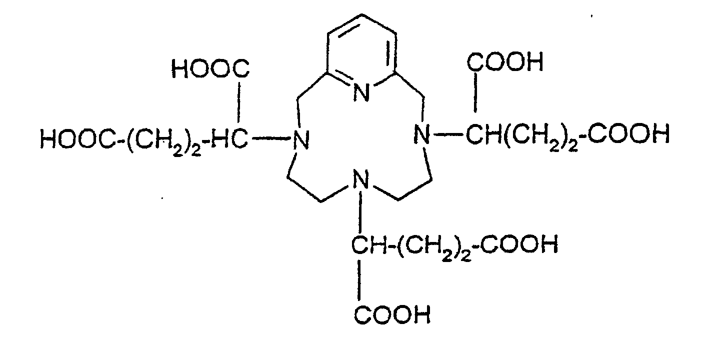 und seine Salze mit einer anorganischen oder organischen Säure.

18. Chelat nach Anspruch 17, worin das Metall Gd ist.

19. Verfahren zur Herstellung der Verbindungen der Formel I nach Anspruch 1, welches darin besteht:
- den Makrocyclus der Formel 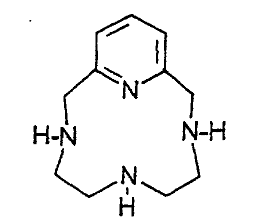 umzusetzen mit einer Verbindung der Formel R'OOC-CHX-(CH₂)₂-COOR', in der X eine austretende Gruppe darstellt, R' H oder (C₁-C₃)-Alkyl bedeutet, und die Esterfunktionen zur hydrolysieren, wenn R' von H verschieden ist,
- dann mit einem Salz oder Oxid eines Metalls umzusetzen zur Bildung des Chelats des gebildeten Produkts oder eines seiner Salze mit einer Base,
- und das Chelat mit dem Amin RNH2 in Gegenwart eines Aktivierungsmittels für die Carbonsäuregruppen umzusetzen.

20. Zubereitung für die diagnostische Bilderzeugung umfassend ein Chelat nach einem der Ansprüche 1 bis 16 und ein pharmazeutisch annehmbares Trägermaterial und gegebenenfalls übliche Formulierungshilfsstoffe.

21. Zubereitung für die Bilderzeugung durch kernmagnetische Resonanz umfassend ein Gadoliniumchelat nach einem der Ansprüche 1 bis 16.
